(19) Europäisches Patentamt
European Patent Office
Office européen des brevets

(11) Veröffentlichungsnummer: **0 361 088 A1**

(12) **EUROPÄISCHE PATENTANMELDUNG**

(21) Anmeldenummer: **89115661.4**

(22) Anmeldetag: **25.08.89**

(51) Int. Cl.[5]: **C07C 229/24 , C07C 227/06 , C11D 1/10 , A01N 37/44**

(30) Priorität: **26.08.88 DE 3829001**

(43) Veröffentlichungstag der Anmeldung: **04.04.90 Patentblatt 90/14**

(84) Benannte Vertragsstaaten: **CH DE ES FR GB IT LI**

(71) Anmelder: **TRIGON CHEMIE GmbH**
**Alte Hohenzeller Strasse 20**
**Ch-6490 Schlüchtern(CH)**

(72) Erfinder: **Bloch, Michael, Dr. rer. nat. Dipl. Chem.**
**Alte Hohenzeller Strasse 20**
**D-6490 Schlüchtern(DE)**

(74) Vertreter: **Eyer, Eckhardt Philipp, Dipl.-Ing. et al**
**Patentanwälte Eyer & Linser**
**Robert-Bosch-Strasse 12a**
**D-6072 Dreieich(DE)**

(54) **Asparaginsäure-Derivate und Verfahren zu ihrer Herstellung.**

(57) Asparaginsäurederivate der Struktur

```
                                  H
                                  !
R - (NH - (CH2)a)b - HN - C - COOX
                                  !
                                  C - COOY
                                  !
                                  H2
```

worin X ein Kation der I. und II. Haupt- und Nebengruppen des Periodischen Systems oder ein Amin, insbesondere Mono-, Di- oder Tri-Äthanolamin,
Y = H oder X sowie
R einen aliphatischen Rest der Struktur $C_nH_{2n}$ mit n = 8 bis 22
oder $CH_3$-$(CH_2)_7$-CH = CH-$(CH_2)_7$-
oder $CH_3(CH_2)_7$-CH(OH)-$CH_2$-$(CH_2)_7$-
oder $CH_3$-$(CH_2)_7$-CH(OH)-CH(OH)-$(CH_2)_7$-
oder $CH_3$-$(CH_2)_5$-CH(OH)-$CH_2$-CH = CH-$(CH_2)_7$-
oder R1-$(O\text{-}A)_k$- mit R1 - R, A einem Alkylrest mit 2 oder 3 Kohlenstoffatomen und k eine Zahl von 1 bis 6
a die Zahl 2 oder 3
b eine Zahl von 0 bis 6
bezeichnen. Ihre Herstellung erfolgt durch Mischen eines primären Amins und eines Monosalzes der Maleinsäure und anschließendes Mischen bei erhöhter Temperatur bis zur vollständigen Umreagierung. Die Reaktion kann bei erhöhtem Druck in wässriger Lösung durchgeführt werden.

EP 0 361 088 A1

## Asparaginsäure-Derivate und Verfahren zu ihrer Herstellung

Gegenstand der Erfindung sind Salze von Asparaginsäurederivaten, ihre Anwendungsprodukte sowie ein Verfahren zu ihrer Herstellung.

Salze von Asparaginsäurederivaten der Strukturen gemäß Patentanspruch 1 sowie ein Verfahren zu ihrer Herstellung sind bisher nicht bekannt. Demgemäß liegt der vorliegenden Erfindung als Aufgabe die Schaffung von Asparaginsäurederivaten und eines Verfahrens zugrunde, das auf produktionstechnisch einfache Weise die Herstellung dieser Substqanzen ermöglicht. Diese Aufgabe wird durch die in Patentanspruch 1 beschriebenen Substanzen bezw. das in Patentanspruch 6 dargestellte Verfahren gelöst.

Es hat sich überraschenderweise gezeigt, daß die aus primären Aminen durch Reaktion mit den Monosalzen der Maleinsäure gewonnenen Monosalze von Asparaginsäurederivaten aufgrund ihrer Wasserlöslichkeit in wässrigem Medium und daher auf herstellungstechnisch außerordentlich einfachem Wege hergestellt werden können und - neben ausgezeichneten tensidischen und weichmachenden Eigenschaften - über einerseits hervorragende biozide und und andererseits enzymstabilisierende Eigenschaften andererseits verfügen, so daß - etwa in einem flüssigen Waschmittel - eingesetzte Enzyme bei gleichzeitigem Einsatz einer der erfindungsgemäßen Substanzen über einen längeren - im Vergleich zu bekannten Waschmitteln mehrfachen - Zeitraum ihre Wirksamkeit behalten. Ein weiterer wesentlicher Vorteil der erfindungsgemäßen Derivate besteht auch darin, daß sie der Bildung von Rückständen auf dem Gewebe und damit dem bei herkömmlichen Waschmitteln auftretenden Vergrauen entgegenwirken.

Aufgrund der vorstehend beschriebenen Eigenschaften eignen sich die erfindungsgemäßen Substanzen ausgezeichnet zum Einsatz in (flüssigen, festen oder pulvrigen) Wasch-, Reinigungs- und Pflegemitteln, in denen sie bei ausgezeichneter Waschwirkung eine Desinfektionswirkung ausüben und darüberhinaus dem Waschgut bei guter Reinigungswirkung einen weichen Griff verleihen, so daß weitgehend auf die heute nach wie vor weit verbreitete Avivage-Nachspülung verzichtet werden kann. Sie können weiterhin als Additive in Korrosionsschutzmitteln bei der Metallverarbeitung und in Formtrennmitteln eingesetzt werden. Weitere wesentliche Vorteile der erfindungsgemäßen Derivate bestehen auch darin, daß sie einerseits der Bildung von Rückständen auf dem Gewebe und damit dem bei herkömmlichen Waschmitteln auftretenden Vergrauen entgegenwirken und daß sie andererseits die Stabilität von evtl. - in flüssigen Waschmitteln - eingesetzten Enzymen erheblich erhöhen, so daß diese bei Einsatz einer der erfindungsgemäßen Substanzen über einen längeren - im Vergleich zu bekannten Waschmitteln mehrfachen - Zeitraum ihre Wirksamkeit behalten.

Die erfindungsgemäßen Substanzen eigenen sich weiterhin ausgezeichnet zum Einsatz in Desinfektionsmitteln, Konservierungsmitteln und Schädlingsbekämpfungsmitteln, in denen sie bereits bei außerordentlich geringen Konzentrationen voll wirksam sind. So zeigt etwa N-Cocos-propylen-di-amin asparaginsäure-di-natrium-salz eine minimale Hemmkonzentration (MIC) bereits bei etwa 11 ppm gegenüber dem Mikroorganismus Streptococus Faecalis und eine minimale Hemmkonzentration 54 ppm gegenüber dem Mikroorganismus Escherischia Coli. Entsprechend weisen N-Cocos-dipropylentriaminasparaginsäure-di-natrium-salz einen MIC-Wert von etwa 25 ppm gegenüber Streptococus Faecalis und von etwa 221 ppm gegenüber Escherischia Coli sowie N-Talg-tripropylen-tetra-amin-asparaginsäuredinatrium-salz einen MIC-Wert von etwa 100 ppm gegenüber Streptococus Faecalis und von etwa 100 ppm gegenüber Escherischia Coli auf.

Die Substanzen weisen darüberhinaus eine außerordentlich gute Kurzzeitwirkung auf. So zeigt etwa N-Cocos-propylen-di-aminasparaginsäure-dinatrium-salz bei einer Einsatzkonzentration von 0,36 % in Wasser nach nur fünf-minütiger Einwirkungszeit eine Verminderung der Bakterienpopulation um 5 bis 6 Zehnerpotenzen bei z.B. Aspergilus Niger oder Salmonella Cerens. Die Substanzen zeichnen sich durch eine beträchtliche Substantivität, gute Wasserlöslichkeit und sehr niedrige Toxizität aus. Sie sind darüberhinaus biologisch abbaubar, so daß auch bei Übertritt in das Abwasser keine nennenswerte Schädigung der biologischen Reinigungssysteme eintritt.

Die Erfindung ist nachstehend anhand einiger Beispiele näher erläutert:

### Beispiel 1

Es werden 138 g in 800 g Wasser und 337 g Isopropanol gelöstem Maleinsäure-Mono-Natriumsalz in einem 2 l Autoklaven mit Rührer und zwei Tropftrichtern 361 g Cocos-dipropylen-triamin (Trinoram C) zugegeben. Die Mischung erwärmt sich während der Zugabe geringfügig. Die sich anschließende Reaktion wird während einer Zeit von etwa sechs Stunden bei einer Temperatur zwischen 120 und 130° C unter

ständigem Rühren geführt. Es bildet sich eine wässrige Lösung von N-Cocos-dipropylen-triamin-Asparaginsäure-Mono-Natriumsalz, das entweder in dieser Form einsetzbar ist oder mit 100 g 40%-iger Natronlauge zu N-Cocos-dipropylen-triamin-asparaginsäure-Di-Natriumsalz neutralisiert werden kann. Durch Trocknen kann wasserfreies Salz isoliert werden.

Beispiel 2

Es werden 138 g in 830 g Wasser und 332 g Isopropanol gelöstem Maleinsäure-Mono-Natriumsalz in einem 2 l Autoklaven mit Rührer und zwei Tropftrichtern 360 g N-Talg-Tripropylen-tetraamin zugegeben. Die Mischung erwärmt sich während der Zugabe geringfügig. Die sich anschließende Reaktion wird während einer Zeit von etwa sechs Stunden bei einer Temperatur zwischen 120 und 130° C unter ständigem Rühren geführt. Es bildet sich eine wässrige Lösung von N-Talg-Tripropylen-tetraamin-Asparaginsäure-Mono-Natriumsalz, das entweder in dieser Form einsetzbar ist oder mit 100 g 40%-iger Natronlauge zu N-Talg-Tripropylen-tetraamin-asparaginsäure-Di-Natriumsalz neutralisiert werden kann. Durch Trocknen kann wasserfreies Salz isoliert werden.

Beispiel 3

Zur Herstellung eines flüssigen Vollwaschmittels werden die nachstehend wiedergegebenen Substanzen eingesetzt:

| | |
|---|---|
| N-Talg-tripropylen-tetra-amin-asparaginsäure-di-natrium-salz | 8,0 % |
| Isotridecanol-3EO | 2,0 % |
| Natrium-Cumol-sulfonat | 1,6 % |
| Dobanol 25-9 | 3,0 % |
| Dobanol 23-6.5 | 1,0 % |
| Dobanol 23-3 | 1,0 % |
| Acrylsäure-copolymer | 1,2 % |
| Zeolyt-Slurry | 26,5 % |
| Anti-Schaum | 0,2 % |
| Enzym | 0,5 % |
| Wasser | 55,0 % |

Das Primärwaschvermögen des im vorstehenden Beispiel 1 wiedergegebenen Waschmittels wurde im Vergleich mit zwei marktgängigen Flüssigwaschmitteln einer farbmetrischen Untersuchung nach dem LSD-Test (least significant difference) an Gewebe unterzogen, das mit "Standard"-Verschmutzung (Ruß/-Mineralöl), Blut und Hautfett versehen war. Die Ergebnisse sind in der nachstehenden Tabelle wiedergegeben, worin
Rem-E die mittlere Deltaremission des Mittels nach Beispiel 1
Rem-I die mittlere Deltaremission des marktgängigen Mittels 1
Rem-II die mittlere Deltsremission des marktgängigen Mittels 2
SumStr.# die Gesamtvarianz der drei verglichenen Waschmittel
LSD-Wert die kleinste signifikante Differenz
bedeuten.

| 1. LSD-Werte bei Waschvorgang 30° C | | | | | |
|---|---|---|---|---|---|
| Schmutz | Rem-E | Rem-I | Rem-II | SumStr.# | LSD-Wert |
| Standard | 16,43 | 11,70 | 10,95 | 2,64 | 1,49 |
| Hautfett | 20,44 | 17,78 | 15,50 | 7,41 | 2,50 |
| Blut | 23,82 | 17,02 | 18,66 | 4,86 | 2,03 |

| 2. LSD-Werte bei Waschvorgang 60° C | | | | | |
|---|---|---|---|---|---|
| Schmutz | Rem-E | Rem-I | Rem-II | SumStr.# | LSD-Wert |
| Standard | 26,28 | 18,73 | 19,91 | 2,40 | 1,42 |
| Hautfett | 25,55 | 24,22 | 27,40 | 1,29 | 1,04 |
| Blut | 36,19 | 25,27 | 22,30 | 4,91 | 2,04 |

Die vorstehend wiedergegebenen Untersuchungsergebnisse zeigen deutlich bessere Waschergebnisse des erfindungsgemäße Waschmittels in nahezu allen Bereichen im Vergleich zu den herangezogenen marktgängigen Produkte, wobei die wesentlich verbesserten Ergebnisse des Waschmittels gemäß der Erfindung in Bezug auf die Griffigkeit des Gewebes unberücksichtigt geblieben sind. Darüberhinaus zeigt das Waschmittel bakterizide Wirksamkeit.

## Ansprüche

1. Asparaginsäurederivate der Struktur

```
                                     H
                                     !
R - (NH - (CH2)a)b - HN - C - COOX
                                     !
                                     C - COOY
                                     !
                                     H2
```

worin X ein Kation der I. und II. Haupt- und Nebengruppen des Periodischen Systems oder ein Amin, insbesondere Mono-, Di- oder Tri-Äthanolamin,
Y = H oder X sowie
R einen aliphatischen Rest der Struktur $C_nH_{2n}$ mit n = 8 bis 22
oder $CH_3$-$(CH_2)_7$-CH = CH-$(CH_2)_7$-
oder $CH_3$-$(CH_2)_7$-CH(OH)-$CH_2$-$(CH_2)_7$-
oder $CH_3$-$(CH_2)_7$-CH(OH)-CH(0H)-$(CH_2)_7$-
oder $CH_3$-$(CH_2)_5$-CH(OH)-$CH_2$-CH = CH-$(CH_2)_7$-
oder R1-$(O\text{-}A)_k$- mit R1 = R, A einem Alkylrest mit 2 oder 3 Kohlenstoffatomen und k eine Zahl von 1 bis 6
a die Zahl 2 oder 3
b eine Zahl von 0 bis 6
bezeichnen.

2. Waschmittel unter Verwendung von Asparaginsäurederivaten nach Anspruch 1, dadurch gekennzeichnet, daß die Asparaginsäurederivate einzeln oder zu mehreren in dem Waschmittel in einer Gesamtmenge zwischen 5 und 80 Gew.-%, vorzugsweise zwischen 20 und 70 Gew.-%, bezogen auf die Gesamtmenge an waschaktiver Substanz enthalten sind.

3. Waschmittel nach Anspruch 2, dadurch gekennzeichnet, daß der in dem Asparaginsäurederivat enthaltene aliphatische Rest eine Kettenlänge von n = 14 bis 18 aufweist.

4. Waschmittel nach Anspruch 1 oder 2, gekennzeichnet durch einen Gehalt an einem Enzym.

5. Biozides Mittel unter Verwendung von Asparaginsäurederivaten nach Anspruch 1, dadurch gekennzeichnet, daß die Asparaginsäurederivate einzeln oder zu mehreren bis zu einer Konzentration von 100%, vorzugsweise in einer Konzentration bis zu 5 Gew.-% enthalten sind.

6. Biozides Mittel nach Anspruch 5, dadurch gekennzeichnet, daß der in dem Asparaginsäurederivat enthaltene aliphatische Rest eine Kettenlänge von n = 8 bis 16 aufweist.

7. Verfahren zur Herstellung von Asparaginsäurederivaten der Strukturen gemäß Anspruch 1, dadurch gekennzeichnet, daß ein primäres Amin mit einem Monosalz der Maleinsäure gemischt werden und die Mischung bei erhöhter Temperatur, vorzugsweise einer Arbeitstemperatur zwischen 100° und 150° C sowie unter erhöhtem Druck, vorzugsweise einem Druck zwischen 2 und 4 atü bis zur vollständigen Umreagierung gerührt wird.

8. Verfahren nach Anspruch 7, dadurch gekennzeichnet, daß die Reaktion in wässriger Lösung erfolgt derart, daß das Maleinsäure-Monosalz in Wasser gelöst und das primäre Amin der Lösung kontinuierlich zugemischt wird.

9. Verfahren nach Anspruch 8, dadurch gekennzeichnet, daß die Reaktion in einer einen chemischen Lösungsvermittler, beispielsweise Isopropanol enthaltenden wässrigen Lösung erfolgt.

Europäisches Patentamt

# EUROPÄISCHER RECHERCHENBERICHT

Nummer der Anmeldung

EP 89115661.4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| X | CHEMICAL ABSTRACTS, Band 64, Nr. 13, 20. Juni 1966, Columbus, Ohio, USA H.J. HUECK et al. "Bacteriostatic, fungistatic and algistatic activity of fatty nitrogen compounds" Spalte 20545, Zusammenfassung-Nr. 20 545b & Appl. Microbiol. 14(3), 308-19(1966) + Chemical Abstracts, Seventh Collective Index, Formulas, Seiten 4483F, linke Spalte, Zeile 31 von unten; Seite 3945, rechte Spalte, Zeile 48 -- | 1,5,6 | C 07 C 229/24<br>C 07 C 227/06<br>C 11 D 1/10<br>A 01 N 37/44 |
| X | CHEMICAL ABSTRACTS, Band 66, Nr. 10, March 6, 1967, Columbus, Ohio, USA SHINICHI TOMIYAMA et al. "Antistatic agents for resins" Seite 5319, Zusammenfassung-Nr. 56 138f & Jan. 10, 1967, Appl. Feb. 19, 1962, and Dec. 2, 1963 -- | 1 | RECHERCHIERTE SACHGEBIETE (Int. Cl 4)<br>C 07 C 229/00<br>C 07 C 227/00<br>C 11 D |
| X | CHEMICAL ABSTRACTS, Band 91, Nr. 8, August 20, 1979, Columbus, Ohio, USA SCHAURICH, K. et al. "Influencing surfactant properties of alkylated aminopolycarboxylic acids by varying the structure" Seite 124, Zusammenfassung-Nr. 59 210a & Tr. - Mezhdunar, Kongr. | 1,2 | |

Der vorliegende Recherchenbericht wurde für alle Patentansprüche erstellt.

| Recherchenort | Abschlußdatum der Recherche | Prüfer |
|---|---|---|
| WIEN | 27-11-1989 | KÖRBER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1503 03 82

Europäisches Patentamt

# EUROPÄISCHER RECHERCHENBERICHT

Nummer der Anmeldung

EP 89115661.4

| Kategorie | EINSCHLÄGIGE DOKUMENTE — Kennzeichnung des Dokuments mit Angabe, soweit erforderlich der maßgeblichen Teile | Betrifft Anspruch | KLASSIFIKATION DER ANMELDUNG |
|---|---|---|---|
| | Poverkhn.-Akt. Veshchestvam, 7th 1976 -- | | |
| X | BEILSTEINS "Handbuch der organischem Chemie", 4. Auflage, 4. Band, Drittes Ergänzungswerk SPRINGER VERLAG, Berlin Seite 1525 * Seite 1525, 5. Absatz von unten * -- | 1,2 | |
| A | CHEMICAL ABSTRACTS, Band 74, Nr. 19, 10. Mai 1971, Columbus, Ohio, USA V.E.LIMANOV et al. "Synthesis and antibacterial properties of ampholytic preparations based on dodecylamine" Seite 545, Zusammenfassung--Nr. 100 387c + Chemical Abstracts, Eight Collective Index, Formulas, Seite 8177F, mittlere Spalte, Formel C20H41N3O4; Seite 7351F, mittlere Spalte, Zeilen 20,21 -- | 1,5,6 | |
| A | DE - B2 - 2 222 899 (H.M.MARUMO) * Anspruch 1; Spalte 22, Verbindung 50 * -- | 1-3 | RECHERCHIERTE SACHGEBIETE |
| A | AT - B - 290 489 (TH.GOLDSCHMIDT A.G.) * Anspruch 1; Beispiel 3 * -- | 1,5,7-9 | |
| X | CHEMICAL ABSTRACTS, Band 64, Nr. 6, 14. März 1966, Columbus, Ohio, USA | 1 | |

Der vorliegende Recherchenbericht wurde für alle Patentansprüche erstellt.

| Recherchenort | Abschlußdatum der Recherche | Prüfer |
|---|---|---|
| WIEN | 27-11-1989 | KÖRBER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

Europäisches Patentamt

# EUROPÄISCHER RECHERCHENBERICHT

Nummer der Anmeldung

-3-

EP 89115661.4

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
| | HOECHST AG "Antistatic additives for polymers" Spalte 8416, Zusammenfassung--Nr. 8 416b & BE-A-641 487 + Chemical Abstracts, Seventh Collective Index, Formulas, Seite 4981F, rechte Spalte, 5. Zeile von unten ---- | | |
| | | | RECHERCHIERTE SACHGEBIETE (Int. Cl.4) |

Der vorliegende Recherchenbericht wurde für alle Patentansprüche erstellt.

| Recherchenort | Abschlußdatum der Recherche | Prüfer |
|---|---|---|
| WIEN | 27-11-1989 | KÖRBER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument